# FASCICULE DE BREVET EUROPEEN

(11) **EP 3 531 389 B1**
(45) Date de publication et mention de la délivrance du brevet: **08.04.2020**
(21) Numéro de dépôt: 19155116.7
(22) Date de dépôt: 01.02.2019
(51) Int. Cl.: G08B 21/06

(54) **PROCEDE DE MESURE DE L'ETAT DE VIGILANCE D'UN PILOTE**
MESSVERFAHREN DES WACHSAMKEITZUSTANDS EINES PILOTEN/FAHRERS
METHOD FOR MEASURING THE STATE OF VIGILANCE OF A PILOT

(30) Priorité: 23.02.2018 FR 1851572
(43) Date de publication de la demande: 28.08.2019
(73) Titulaire: AIRBUS OPERATIONS (S.A.S.), 31060 Toulouse (FR)
(72) Inventeur: CLEMENT, Nicolas, 31200 Toulouse (FR); GAUDUIN, Benoît, 31300 Toulouse (FR)
(74) Mandataire: Gicquel, Olivier Yves Gérard

(56) Documents cités:
- EP-A2- 2 434 465
- WO-A1-92/07346
- US-A- 5 339 259
- US-A1- 2015 104 038

## Description

La présente invention concerne un procédé de mesure de l'état de vigilance d'un pilote au pilotage d'un aéronef.

L'incapacité d'un pilote au pilotage un aéronef se traduit par un comportement anormal du pilote pendant plusieurs secondes, en particulier marqué par un manque de vigilance du pilote qui peut être dû à un malaise physique ou psychologique (stress). Dès lors qu'une suspicion d'incapacité au pilotage existe, un plan d'action est initié pour remplacer ou assister le pilote afin de poursuivre le vol de l'aéronef dans des conditions optimales.

Durant un vol, l'état de vigilance d'un pilote est mesuré par un autre pilote, le contrôle au sol avec lequel le pilote est en contact radio permanent, ou encore, comme décrit dans la demande de brevet EP2434465, par des capteurs portés par le pilote (capteurs cardiaques par exemple). Le port de tels capteurs durant plusieurs heures de vol peut incommoder les pilotes.

La présente invention a pour objectif de proposer un moyen de mesurer l'état de vigilance d'un pilote qui ne soit incommodant pour le pilote. A cet effet, l'invention concerne un procédé de mesure de l'état de vigilance d'un pilote d'un aéronef qui est revendiqué dans la revendication 1.

Le procédé selon l'invention est non intrusif pour le pilote et permet de mesurer l'état de vigilance d'un pilote et de lancer une alerte dans le cas où le pilote manque de vigilance.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
- la figure 1 représente un aéronef comportant un dispositif de mesure pour la mise en œuvre du procédé de mesure de l'état de vigilance d'un pilote selon un mode de réalisation de l'invention ;
- la figure 2 représente une vue d'un poste de pilotage de l'aéronef de la figure 1 dans lequel sont arrangés différents haut-parleurs utilisés dans la mise en œuvre du procédé de mesure de l'état de vigilance d'un pilote selon un mode de réalisation de l'invention ;
- la figure 3 est une vue d'un casque d'un pilote utilisé dans la mise en œuvre du procédé de mesure de l'état de vigilance d'un pilote selon un mode de réalisation de l'invention ; et
- la figure 4 est un diagramme représentant les différentes étapes du procédé de mesure de l'état de vigilance d'un aéronef selon un mode de réalisation de l'invention.

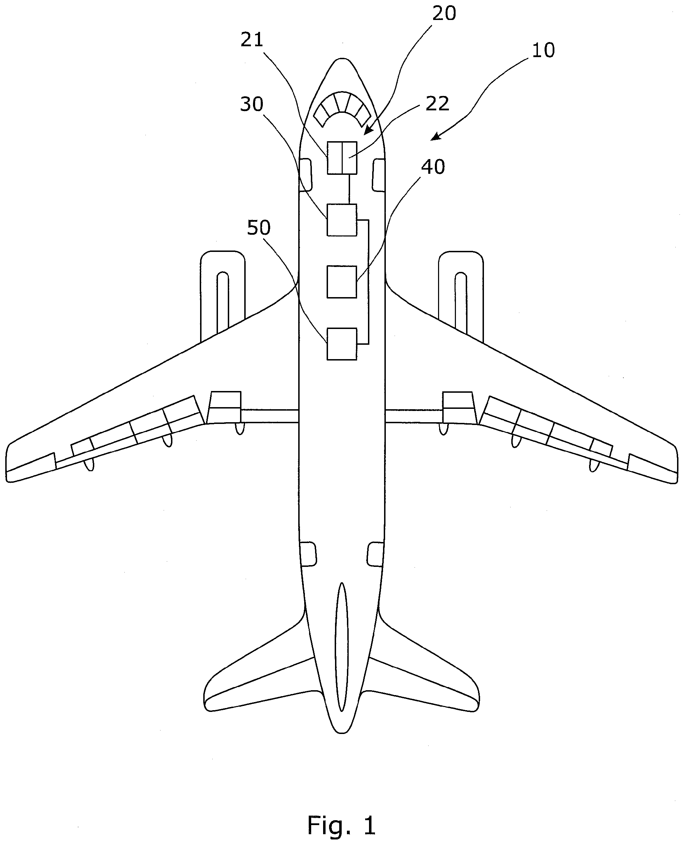

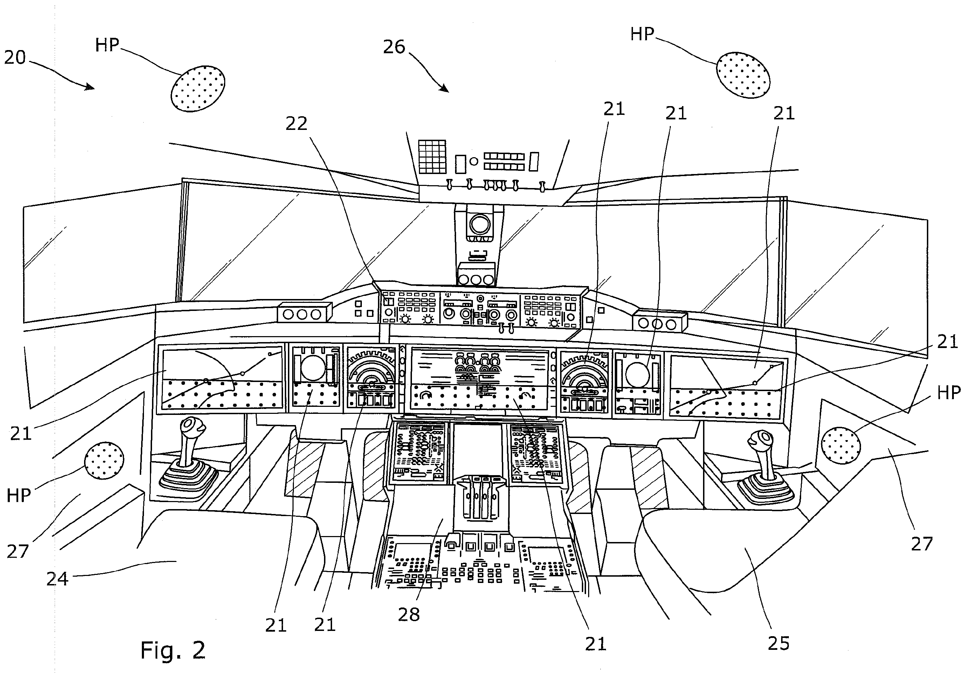

En référence avec les figures 1 et 2, un aéronef 10 comprend un poste de pilotage 20 dans lequel sont installés les sièges d'un premier et d'un second pilote (copilote) 24,15, différents écrans 21 et une interface homme-machine 22 (les écrans comprennent une interface homme-machine lorsqu'ils sont tactiles) avec lequel un pilote peut interagir pour donner des instructions aux composants de l'aéronefs.

L'aéronef comprend en outre des moyens de communications qui se composent d'une unité de communication audio 30 (Audio Management Unit) de type unité centrale ayant pour fonction de gérer les communications de l'aéronef, et qui est connectée pour cela à des antennes (non représentées) situées sur le fuselage de l'aéronef pour la réception ou l'émission de signaux électriques, à un système d'alerte 50 qui émet des signaux électriques d'alerte vers au mois un écran 21 dans le cas de la survenue d'une anomalie (exemple : alarme de décrochage, alarme incendie, ...), et à des transducteurs pour convertir des signaux électriques en ondes sonores ou inversement.

Les transducteurs sont arrangés dans le poste de pilotage 20 et comprennent :
- au moins deux haut-parleurs HP, par exemple agencés sur le plafond 26 ou sur des consoles 27,28 du poste de pilotage 20;
- pour chaque pilote, un microphone M et deux écouteurs 31,32 arrangés sur le casque C (boomset en anglais) de chaque pilote.

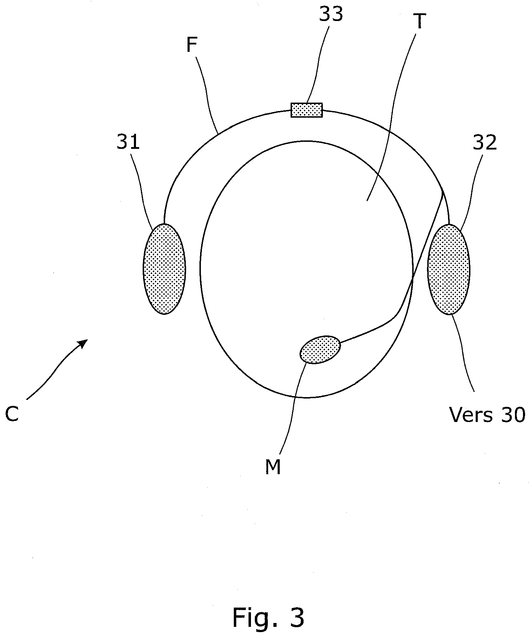

De manière connue, et en référence avec la figure 3, un casque C comprend un arceau F à chaque extrémité duquel est fixé un écouteur 31,32. Le microphone M est fixé à l'arceau F tout en étant déporté de ce dernier. En position d'utilisation, le pilote règle le casque de sorte que l'arceau F enveloppe le haut de sa tête T, avec un écouteur 31,32 situé devant chaque oreille, le microphone M étant lui situé devant la bouche du pilote.

Selon l'invention, l'aéronef 10 comprend un dispositif de mesure de l'état de vigilance d'un pilote de l'aéronef 40, dit dispositif de mesure, qui est configuré pour mesurer le temps entre lequel une onde sonore est émise par un haut-parleur HP et est reçue par le microphone M d'un casque et calculer les coordonnées d'une sphère centrée sur le haut-parleur HP et sur le périmètre de laquelle le microphone M se situe.

Le calcul des points d'intersections d'au moins deux sphères centrées sur des haut-parleurs HP différents fournit des positions possibles du microphone M dans l'espace du poste de pilotage 20. Ces positions sont celles de la tête du pilote T lorsque le casque C est utilisé par ce dernier.

Via la connaissance d'au moins une position possible de la tête T du pilote dans le poste de pilotage 20, le dispositif de mesure 40 est configuré pour mesurer l'état de vigilance d'un pilote de l'aéronef 10.

Le dispositif de mesure 40 est soit un module, intégré dans l'unité de communication audio 30, soit un module de type unité centrale, distinct de l'unité de communication audio 30 (comme illustré par la figure 1), et connecté à cette dernière ainsi qu'au système d'alerte 50.

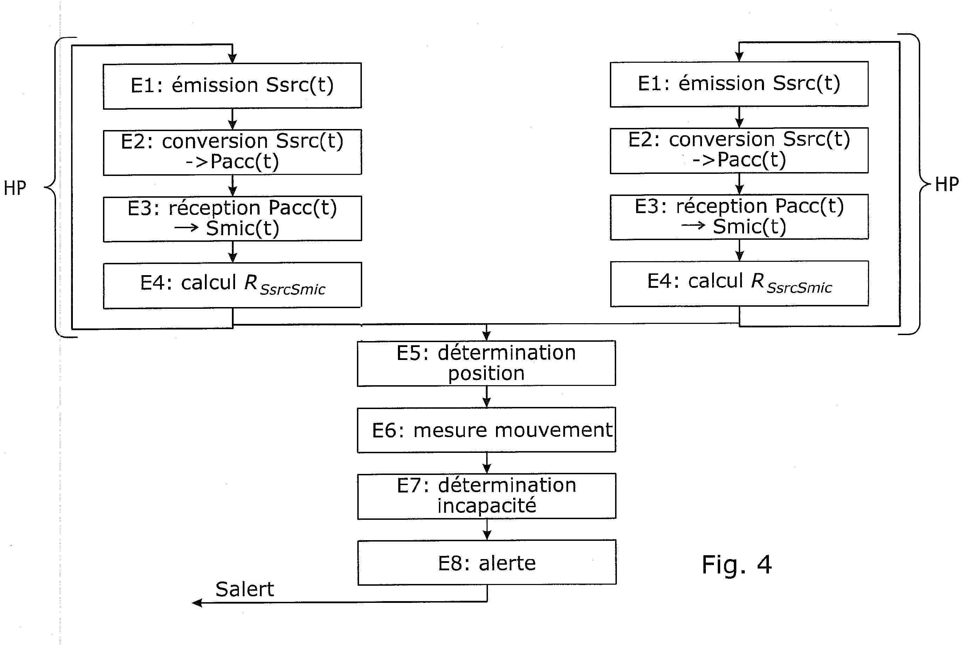

En référence avec la figure 4, le procédé de mesure de l'état de vigilance d'un pilote mis en œuvre par le dispositif de mesure 40 va être explicité pour sa mise en œuvre dans un poste de pilotage d'aéronef 20 comprenant deux hauts parleurs HP. Un haut-parleur HP dédié au premier pilote est arrangé à proximité du siège 24 de ce dernier, et de manière symétrique selon un plan médian du fuselage de l'aéronef, un autre haut-parleur HP dédié au second pilote (copilote) est arrangé à proximité du siège 25 de ce dernier.

On considère uniquement le cas du microphone M du casque C du premier pilote, mais le procédé tel que décrit s'applique de manière identique pour chaque microphone M du pilote/copilote puisque les entrées des microphones M sont différenciées au niveau de l'unité de communication audio 30 de sorte que ladite unité peut différencier les signaux qu'elle reçoit des microphones M.

Dans une étape E1 d'émission, l'unité de communication audio 30 émet un signal d'excitation électrique Ssrc(t) vers un haut-parleur HP. Un signal d'excitation électrique émis par l'unité de communication audio vers un haut-parleur est par exemple :
- un signal reçu de l'extérieur de l'aéronef (autre aéronef, sol...) via une antenne ;
- un signal électrique d'alerte généré par le système d'alerte 50. Le signal électrique d'alerte est, par exemple, un signal, qui lorsqu'il sera convertit en onde acoustique, sera un bip, une tonalité, une alarme ; et
- un signal spécifique à la mesure de l'état de vigilance d'un pilote, qui est généré par le dispositif de mesure 40. Le signal spécifique est, par exemple, configuré pour être inaudible par les pilotes une fois convertit en onde acoustique.

Dans une étape E2 de conversion, le haut-parleur HP convertit le signal d'excitation Ssrc(t) en onde acoustique Pacc(t), puis dans une étape E3 de réception, le microphone M du casque C d'un pilote capte l'onde acoustique Pacc(t), et la convertit en signal électrique Smic(t) qui est transmise vers l'unité de communication audio 30.

A l'issue de l'étape E3, dans une étape E4 de calcul, le dispositif de mesure 40 calcule la distance du haut-parleur HP au microphone M du casque du pilote et calcule les coordonnées d'une sphère autour du haut-parleur HP dans sur le périmètre de laquelle le microphone se situe.

Dans le détail, le calcul de la distance et des coordonnées de la sphère comprend :
- a) le calcul du maximum de la fonction d'intercorrélation *R_{SsrcSmic}* entre le signal d'excitation électrique Ssrc(t) et le signal électrique Smic(t) émis par un microphone ayant reçu l'onde acoustique Pacc(t) du haut-parleur HP, le maximum ainsi calculé représente le temps de propagation acoustique du signal d'excitation Ssrc1(t).
- b) le calcul de la distance du haut-parleur HP au microphone M, en multipliant le temps de propagation calculé par la célérité du son ; et
- c) la détermination des coordonnées de la sphère, rendue possible par le fait que haut-parleur HP a une position fixe et connue dans le poste de pilotage 20 (la position est enregistrée dans une mémoire du dispositif de mesure 40).

Les quatre étapes E1-E4 sont mises en œuvre pour l'autre haut-parleur HP. De manière générale, les quatre étapes E1-E4 sont mises en œuvre pour un haut-parleur à chaque fois qu'un signal d'excitation électrique Ssrc(t) est émis par l'unité de communication audio 30 vers ledit haut-parleur HP.

En fonction de la nature des signaux d'excitation électrique Ssrc(t), les quatre étapes E1-E4 sont mises en œuvre simultanément sur chacun des hauts parleurs HP (cas dans le cas d'une alerte ou d'une signal radio), ou peuvent être mises en œuvre de manière décalée lorsque les signaux d'excitation électrique Ssrc(t) sont des signaux spécifiques à la mesure de l'état de vigilance.

Dans une étape E5 de détermination de la position de microphone M mise en œuvre lorsque les coordonnées de deux sphères centrées sur deux haut-parleurs HP différents ont été calculées (c'est-à-dire à chaque fois qu'un cycle d'étapes E1 à E4 est réalisé pour un premier et un second haut-parleur HP), le dispositif de mesure 40 calcule les points d'intersection des sphères situées autour des deux haut-parleurs HP. Ces points d'intersections sont des positions possibles du microphone M dans l'espace du poste de pilotage.

Dans une étape E6 de mesure du mouvement du microphone, le dispositif de mesure 40 mesure, pendant une durée prédéterminée (par exemple, de l'ordre de 30s), les mouvements du microphone M en prenant pour référence une des positions possibles, dite position de détection, du microphone M, déterminée à l'étape E5 précédente. On notera que l'augmentation de la fréquence d'émission de signaux d'excitation électriques pour chaque haut-parleur augmente la précision de la mesure du mouvement du microphone.

Ensuite, dans une étape E7 de détermination d'incapacité, le dispositif de mesure 40 détermine que le pilote présente un manque de vigilance si le microphone M ne s'est pas éloigné de la position de détection d'une distance prédéterminée (de l'ordre de quelques centimètres, 2 cm par exemple) pendant la durée prédéterminée.

Si durant la durée prédéterminée, la tête du pilote s'est éloignée de la position de détection de la distance prédéterminée, alors le dispositif de mesure 40 reprend la mesure de l'état de vigilance à l'étape E5.

Au contraire, si durant la durée prédéterminée, la tête du pilote ne s'est pas éloignée de la position de détection de la distance prédéterminée, alors le dispositif de mesure 40, dans une étape E8 d'alerte, génère un signal électrique Salert indicatif d'un manque de vigilance du pilote.

Ce signal Salert est transmis à l'unité de communication audio 30 pour l'envoi d'un signal aux écouteurs du casque C du pilote pour lequel le dispositif de mesure 40 a mesuré un état de manque de vigilance. Le signal est dans ce cas prévu pour être transformé en son d'alarme audible par les écouteurs 31,32 du casque C du pilote et l'alarme est émise tant que le pilote n'accomplit pas une action prédéfinie, comme par exemple un mouvement de la tête, une interaction spécifique avec l'interface homme-machine 22, ....

En variante, le signal Salert est transmis au système d'alerte 50 pour l'affichage d'un message sur au moins l'un des écrans 21 du poste de pilotage 20. Le message est ostensible (par exemple clignotant, d'une couleur vive...) et comporte une instruction demandant au pilot/copilote d'accomplir une action (interaction demandée au pilote avec l'interface homme-machine 22) dans un délai prédéterminé (par exemple 30 secondes) afin de s'assurer qu'au moins un pilote est apte au pilotage. Si le pilote ou le copilote accomplit l'action demandée dans le délai, le dispositif de mesure 40 reprend la mesure de l'état de vigilance. En revanche si aucun pilote n'accomplit l'action, un signal est envoyé par le dispositif de mesure 40 vers une assistance au sol pour la mise en œuvre de mesures d'assistance, comme par exemple la prise de contrôle de l'aéronef à distance.

L'invention est particulièrement adaptée à une configuration de poste de pilotage 20 qui comprend plus de deux haut-parleurs HP. Si le poste de pilotage comprend un nombre n de haut-parleurs HP, les étapes E1 à E4 peuvent être effectuées pour chacun des n haut-parleurs. Puisque le nombre de points d'intersections de n sphères diminue à mesure que le nombre de haut-parleurs est important, la précision de la détermination de la position du microphone M est d'autant plus importante que le nombre de haut-parleurs HP est élevé. Ainsi, lorsque le poste de pilotage 20 comprend au moins 4 haut-parleurs, l'intersection de chacune des quatre sphères est un unique point.

Dans la description du procédé décrite ci-dessus, on a considéré que le pilote porte son casque C durant tout le vol, par exemple du fait d'une obligation formulée par sa compagnie aérienne. En variante, le dispositif de mesure 40 est configuré pour prendre en compte le fait que pour de longues durées de vol, le pilote peut avoir à enlever son casque C.

A cette fin, dans un mode de réalisation de l'invention, le casque C du pilote comprend un capteur de proximité 33 monté sur l'arceau F du casque et dont la zone de détection est orientée vers la partie concave du casque. Le capteur 33 génère un signal Sactiv comprenant l'information selon laquelle le casque C est porté par le pilote lorsque la tête T du pilote se situe à proximité du capteur 33 (par exemple, moins de deux centimètres). En considérant un exemple dans une logique Booléenne où le signal Sactiv mis à 1 indique que la casque C est porté par le pilote, le dispositif de mesure40 recevant la sortie du capteur est apte à générer un signal électrique Salert uniquement lorsque le signal Sactiv est mis à 1

La description du procédé tel que décrit précédemment est alors modifiée en ce sens :
- qu'il comprend une étape EP de vérification de l'utilisation du casque C par le pilote, mis en œuvre en permanence par le dispositif de mesure 40, dans laquelle le dispositif de mesure 40 vérifie que le casque est porté par le pilote (réception d'un signal Sactiv mis à 1 en logique booléen considérée ici à titre d'exemple uniquement), ou n'est pas porté par le pilote (réception d'un signal Sactiv mis à 0).
- que l'étape E8 d'alerte est mise en œuvre à la condition supplémentaire que le casque C est porté par le pilote (soit Sactiv mis à 1).

Dans un mode de réalisation préférentiel de l'invention, lorsque les signaux d'excitations électrique Ssrc(t) sont des signaux spécifiques à la mesure de l'état de vigilance d'un pilote, l'unité de communication audio 30 génère un signal d'excitation électrique distinct pour chaque haut-parleur HP et ces signaux sont envoyées vers les différents haut-parleurs HP en décalé Il est ainsi possible de différencier chaque signal émis par un haut-parleur. Selon ce mode de réalisation, le mouvement du microphone est mesuré en continu et la mesure de l'état de vigilance est plus précise.

## Revendications

1. Procédé de mesure de l'état de vigilance d'un pilote d'un aéronef (10), l'aéronef comprenant un poste de pilotage (20) dans lequel sont arrangés au moins un écran (21) et une interface-homme machine (22), ainsi qu'au moins deux haut-parleurs (HP), et au moins un casque (C) comprenant un microphone (M) prévu pour être utilisé par un pilote et au moins un écouteur (31,32), le microphone (M) étant situé à proximité de la tête (T) du pilote lorsque ce dernier utilise son casque (C), l'aéronef (10) comprenant en outre une unité de communication audio (30) configurée pour gérer les communications de l'aéronef (10) et à laquelle sont connectés les haut-parleurs (HP) et le casque (C), ainsi qu'un dispositif de mesure (40) de l'état de vigilance d'un pilote de l'aéronef, dit dispositif de mesure, connecté à l'unité de communication audio (30) et configuré pour mesurer le temps entre lequel une onde sonore est émise par un haut-parleur (HP) et est reçue par le microphone (M) et en calculer une pluralité de positions possibles du microphone (M) autour du haut-parleur (HP), le procédé comprenant les étapes suivantes :
- une étape E1 d'émission dans laquelle l'unité de communication audio (30) émet un signal d'excitation électrique (Ssrc(t)) vers un haut-parleur (HP) ;
- une étape E2 de conversion dans laquelle le haut-parleur (HP) convertit le signal d'excitation (Ssrc(t)) en onde acoustique (Pacc(t)) ;
- une étape E3 de réception dans laquelle le microphone (M) capte l'onde acoustique (Pacc(t)) et la convertit en signal électrique (Smic(t)) transmis vers l'unité de communication audio (30) ;
- une étape E4 de calcul, dans laquelle le dispositif de mesure (40) calcule la distance du haut-parleur (HP) au microphone (M) et calcule les coordonnées d'une sphère centrée sur le haut-parleur (HP) et sur le périmètre de laquelle le microphone (M) se situe ;
les quatre étapes E1-E4 étant mises en œuvre pour chaque haut-parleur (HP) à chaque fois qu'un signal d'excitation électrique (Ssrc(t)) est émis par l'unité de communication audio (30) vers ledit haut-parleur,
- une étape E5 de détermination de la position du microphone (M) mise en œuvre par le dispositif de mesure (40) lorsque les coordonnées d'au moins deux sphères centrées sur des haut-parleurs (HP) différents ont été calculées, et dans laquelle le dispositif de mesure (40) calcule les points d'intersection des sphères, lesdits points d'intersection étant des positions possibles du microphone et étant représentatives des positions possibles de la tête (T) du pilote lorsque ce dernier utilise son casque ;
- une étape E6 de mesure du mouvement du microphone (M), dans laquelle le dispositif de mesure (40) mesure, pendant une durée prédéterminée, les mouvements d'au moins une position possible du microphone (M), dite position de détection, déterminée à l'étape E5 précédente ;
- une étape E7 de détermination de manque de vigilance, dans laquelle le dispositif de mesure (40) détermine que le pilote présente un manque de vigilance si le microphone (M) ne s'est pas éloigné de la position de détection d'une distance prédéterminée pendant la durée prédéterminée ;
- une étape E8 d'alerte, mise en œuvre dans le cas où le dispositif de mesure (40) a déterminé que le pilote présente un manque de vigilance, dans laquelle le dispositif de mesure (40) génère un signal électrique (Salert) indicatif d'un manque de vigilance ;
dans l'étape E4 de calcul mise en œuvre pour un haut-parleur (HP), le dispositif de mesure (40) :
a) calcule le maximum de la fonction d'intercorrélation (*R_{SsrcSmic}*) entre le signal d'excitation électrique (Ssrc(t)) de l'unité de communication audio (30) et le signal électrique (Smic(t)) provenant du microphone (M), le maximum ainsi calculé représentant le temps de propagation acoustique du signal d'excitation électrique ;
b) calcule la distance du haut-parleur (HP) au microphone (M); et
c) détermine les coordonnées de la sphère autour du haut-parleur (HP).

2. Procédé de détection selon la revendication 1, **caractérisé en ce que** suite à l'étape E8 d'alerte, le signal électrique (Salert) indicatif d'un manque de vigilance du pilote est transmis à l'unité de communication audio (30), et **en ce qu'**a la réception dudit signal électrique (Salert), l'unité de communication audio (30) émet un signal d'excitation électrique vers au moins un écouteur (31,32) du casque (C) pour l'émission d'un son d'alarme audible par le pilote.

3. Procédé de détection selon la revendication 1, l'aéronef (10) comprenant un système d'alerte (50) configuré, dans le cas de la survenue d'une anomalie, pour émettre des signaux électriques d'alerte et afficher des messages d'alerte sur ledit au moins un écran (21), **caractérisé en ce que** suite à l'étape E8 d'alerte, le signal électrique (Salert) indicatif d'un manque de vigilance du pilote est transmis au système d'alerte (50) pour l'affichage, sur le au moins un écran (21), d'un message demandant au pilote d'effectuer une action via l'interface homme-machine (22) dans un délai prédéterminé, et
- si un pilote accomplit l'action dans le délai, le dispositif de mesure (40) reboucle à l'étape (E5) de détermination de la position du microphone (M)
- si un pilote n'accomplit pas l'action dans le délai, un signal est envoyé par le dispositif de mesure (40) vers une assistance au sol pour la mise en œuvre de mesures d'assistance.

4. Procédé de détection selon l'une quelconque des revendications 1 à 3, le casque (C) comprenant un arceau (F) destiné à enserrer la tête (T) du pilote lorsque le casque est utilisé, et un capteur de proximité (33) monté sur l'arceau (F), une zone de détection dudit capteur (33) étant orientée vers une partie concave dudit arceau, le capteur (C) étant configuré pour générer un signal (Sactiv) comprenant l'information selon laquelle le casque (C) est utilisé lorsque la tête (T) du pilote est située en deçà d'une distance prédéterminée du capteur (33),
**caractérisé en ce que** le procédé comprend une étape EP de vérification de l'utilisation du casque (C) par le pilote, mis en œuvre en permanence par le dispositif de mesure (40), dans laquelle le dispositif de mesure (40) détermine si le casque (C) est utilisé ou non par le pilote en fonction de l'information contenue dans le signal (Sactiv) généré par le capteur (33),
et **en ce que** l'étape E8 d'alerte est mise en œuvre à la condition supplémentaire que le casque (C) est utilisé par le pilote.

5. Procédé de détection selon l'une quelconque des revendications 1 à 4, l'aéronef (10) comprenant un système d'alerte (50) configuré, dans le cas de la survenue d'une anomalie, pour émettre des signaux électriques d'alerte et afficher des messages d'alerte sur ledit au moins un écran (21), **caractérisé en ce qu'**un signal d'excitation électrique émis par l'unité de communication audio vers un haut-parleur est un signal pris parmi :
- un signal reçu de l'extérieur de l'aéronef (10) ;
- un signal électriques d'alerte généré par le système d'alerte (50) ;
- un signal spécifique à la détection de l'incapacité d'un pilote, généré par le dispositif de mesure (40).

## Patentansprüche

1. Verfahren zur Messung des Wachsamkeitszustands eines Piloten eines Luftfahrzeugs (10), wobei das Luftfahrzeug ein Cockpit (20) enthält, in dem mindestens ein Bildschirm (21) und eine Mensch-Maschine-Schnittstelle (22) sowie mindestens zwei Lautsprecher (HP) und mindestens ein Helm (C) angeordnet sind, der ein Mikrofon (M), das vorgesehen ist, um von einem Piloten benutzt zu werden, und mindestens einen Kopfhörer (31, 32) enthält, wobei das Mikrofon (M) sich in der Nähe des Kopfes (T) des Piloten befindet, wenn letzterer seinen Helm (C) benutzt, wobei das Luftfahrzeug (10) außerdem eine Audiokommunikationseinheit (30), die konfiguriert ist, die Kommunikationen des Luftfahrzeugs (10) zu verwalten, und mit der die Lautsprecher (HP) und der Helm (C) verbunden sind, sowie eine Messvorrichtung (40) des Wachsamkeitszustands eines Piloten des Luftfahrzeugs enthält, Messvorrichtung genannt, die mit der Audiokommunikationseinheit (30) verbunden und konfiguriert ist, den Zeitraum zu messen, in dem eine Schallwelle von einem Lautsprecher (HP) gesendet und vom Mikrofon (M) empfangen wird, und daraus eine Vielzahl möglicher Positionen des Mikrofons (M) um den Lautsprecher (HP) herum zu berechnen, wobei das Verfahren die folgenden Schritte enthält:
- einen Sendeschritt E1, in dem die Audiokommunikationseinheit (30) ein elektrisches Erregungssignal (Ssrc(t)) an einen Lautsprecher (HP) sendet;
- einen Umwandlungsschritt E2, in dem der Lautsprecher (HP) das Erregungssignal (Ssrc(t)) in eine Schallwelle (Pacc(t)) umwandelt;
- einen Empfangsschritt E3, in dem das Mikrofon (M) die Schallwelle (Pacc(t)) auffängt und sie in ein elektrisches Signal (Smic(t)) umwandelt, das an die Audiokommunikationseinheit (30) übertragen wird;
- einen Rechenschritt E4, in dem die Messvorrichtung (40) den Abstand des Lautsprechers (HP) zum Mikrofon (M) berechnet und die Koordinaten einer auf den Lautsprecher (HP) zentrierten Kugelfläche berechnet, auf deren Umfang sich das Mikrofon (M) befindet;
wobei die vier Schritte El-E4 für jeden Lautsprecher (HP) jedes Mal durchgeführt werden, wenn ein elektrisches Erregungssignal (Ssrc(t)) von der Audiokommunikationseinheit (30) an den Lautsprecher gesendet wird,
- einen Schritt E5 der Feststellung der Position des Mikrofons (M), der von Messvorrichtung (40) durchgeführt wird, wenn die Koordinaten von mindestens zwei auf unterschiedliche Lautsprecher (HP) zentrierten Kugelflächen berechnet wurden, und in dem die Messvorrichtung (40) die Schnittpunkte der Kugelflächen berechnet, wobei die Schnittpunkte mögliche Positionen des Mikrofons und für die möglichen Positionen des Kopfes (T) des Piloten repräsentativ sind, wenn letzterer seinen Helm benutzt;
- einen Schritt E6 des Messens der Bewegung des Mikrofons (M), in dem die Messvorrichtung (40) während einer vorbestimmten Dauer die Bewegungen mindestens einer möglichen Position des Mikrofons (M), Erfassungsposition genannt, misst, die im vorhergehenden Schritt E5 festgelegt wurde;
- einen Schritt E7 der Feststellung einer mangelnden Wachsamkeit, in dem die Messvorrichtung (40) feststellt, dass der Pilot eine mangelnde Wachsamkeit aufweist, wenn das Mikrofon (M) sich während der vorbestimmten Dauer nicht um eine vorbestimmte Entfernung von der Erfassungsposition entfernt hat;
- einen Schritt E8 des Alarms, der in dem Fall durchgeführt wird, in dem die Messvorrichtung (40) festgestellt hat, dass der Pilot eine mangelnde Wachsamkeit aufweist, in dem die Messvorrichtung (40) ein elektrisches Signal (Salert) erzeugt, das auf eine mangelnde Wachsamkeit hinweist;
im für einen Lautsprecher (HP) durchgeführten Rechenschritt E4 die Messvorrichtung (40):
a) das Maximum der Interkorrelationsfunktion (R_{SsrcSmic}) zwischen dem elektrischen Erregungssignal (Ssrc(t)) der Audiokommunikationseinheit (30) und dem vom Mikrofon (M) kommenden elektrischen Signal (Smic(t)) berechnet, wobei das so berechnete Maximum die akustische Ausbreitungszeit des elektrischen Erregungssignals darstellt;
b) den Abstand des Lautsprechers (HP) zum Mikrofon (M) berechnet; und
c) die Koordinaten der Kugelfläche um den Lautsprecher (HP) berechnet.

2. Erfassungsverfahren nach Anspruch 1, **dadurch gekennzeichnet, dass** nach dem Schritt E8 des Alarms das auf eine mangelnde Wachsamkeit des Piloten hinweisende elektrische Signal (Salert) an die Audiokommunikationseinheit (30) übertragen wird, und dass bei Empfang des elektrischen Signals (Salert) die Audiokommunikationseinheit (30) ein elektrisches Erregungssignal an mindestens einen Kopfhörer (31, 32) des Helms (C) zum Senden eines für den Piloten hörbaren Alarmtons sendet.

3. Erfassungsverfahren nach Anspruch 1, wobei das Luftfahrzeug (10) ein Alarmsystem (50) enthält, das konfiguriert ist, im Fall des Auftretens einer Anomalie elektrische Alarmsignale zu senden und Alarmnachrichten auf dem mindestens einen Bildschirm (21) anzuzeigen, **dadurch gekennzeichnet, dass** nach dem Alarmschritt E8 das auf eine mangelnde Wachsamkeit des Piloten hinweisende elektrische Signal (Salert) an das Alarmsystem (50) zur Anzeige auf dem mindestens einen Bildschirm (21) einer Nachricht übertragen wird, die vom Piloten verlangt, in einem vorbestimmten Zeitraum eine Aktion über die Mensch-Maschine-Schnittstelle (22) auszuführen, und
- wenn ein Pilot die Aktion in dem Zeitraum erledigt, die Messvorrichtung (40) zum Schritt (E5) der Feststellung der Position des Mikrofons (M) zurückschleift,
- wenn ein Pilot die Aktion nicht in dem Zeitraum erledigt, ein Signal von der Messvorrichtung (40) an eine Bodenhilfe zur Durchführung von Hilfsmaßnahmen geschickt wird.

4. Erfassungsverfahren nach einem der Ansprüche 1 bis 3, wobei der Helm (C) einen Bügel (F), der dazu bestimmt ist, den Kopf (T) des Piloten zu umklammern, wenn der Helm benutzt wird, und einen auf den Bügel (F) montierten Näherungssensor (33) enthält, wobei eine Erfassungszone des Sensors (33) zu einem konkaven Teil des Bügels ausgerichtet ist, wobei der Sensor (C) konfiguriert ist, ein Signal (Sactiv) zu erzeugen, das die Information enthält, gemäß der der Helm (C) benutzt wird, wenn der Kopf (T) des Piloten sich diesseits eines vorbestimmten Abstands zum Sensor (33) befindet, **dadurch gekennzeichnet, dass** das Verfahren einen Schritt EP der Überprüfung der Benutzung des Helms (C) durch den Piloten enthält, der durchgehend von der Messvorrichtung (40) durchgeführt wird, wobei die Messvorrichtung (40) abhängig von der in dem vom Sensor (33) erzeugten Signal (Sactiv) enthaltenen Information feststellt, ob der Helm (C) vom Piloten benutzt wird oder nicht,
und dass der Alarmschritt E8 unter der zusätzlichen Bedingung durchgeführt wird, dass der Helm (C) vom Piloten benutzt wird.

5. Erfassungsverfahren nach einem der Ansprüche 1 bis 4, wobei das Luftfahrzeug (10) ein Alarmsystem (50) enthält, das im Fall des Auftretens einer Anomalie konfiguriert ist, elektrische Alarmsignale zu senden und Alarmnachrichten auf dem mindestens einen Bildschirm (21) anzuzeigen, **dadurch gekennzeichnet, dass** ein von der Audiokommunikationseinheit an einen Lautsprecher gesendetes elektrisches Erregungssignal ein Signal ist, das ausgewählt wird unter:
- einem von außerhalb des Luftfahrzeugs (10) empfangenen Signal;
- einem vom Alarmsystem (50) erzeugten elektrischen Alarmsignal;
- einem von der Messvorrichtung (40) erzeugten, für die Erfassung der Unfähigkeit eines Piloten spezifischen Signal.

## Claims

1. Method of measuring the state of vigilance of a pilot of an aircraft (10), the aircraft comprising a piloting station (20) in which are arranged at least one screen (21) and a man-machine interface (22), as well as least two loudspeakers (HP), and at least one headset (C) comprising a microphone (M) designed to be used by a pilot and at least one earpiece (31, 32), the microphone (M) being situated in proximity to the head (T) of the pilot when the latter uses his headset (C), the aircraft (10) furthermore comprising an audio communication unit (30) configured to manage the communications of the aircraft (10) and to which are connected the loudspeakers (HP) and the headset (C), as well as a device (40) for measuring the state of vigilance of a pilot of the aircraft, termed the measuring device, connected to the audio communication unit (30) and configured to measure the time between which a sound wave is emitted by a loudspeaker (HP) and is received by the microphone (M) and to calculate a plurality of possible positions of the microphone (M) around the loudspeaker (HP), the method comprising the following steps:
- a step E1 of emission in which the audio communication unit (30) emits an electrical excitation signal (Ssrc(t)) to a loudspeaker (HP) ;
- a step E2 of conversion in which the loudspeaker (HP) converts the excitation signal (Ssrc(t)) into an acoustic wave (Pacc(t));
- a step E3 of reception in which the microphone (M) captures the acoustic wave (Pacc(t)) and converts it into an electrical signal (Smic(t)) transmitted to the audio communication unit (30) ;
- a step E4 of calculation, in which the measuring device (40) calculates the distance from the loudspeaker (HP) to the microphone (M) and calculates the coordinates of a sphere centred on the loudspeaker (HP) and on the perimeter of which the microphone (M) is situated;
the four steps E1-E4 being implemented for each loudspeaker (HP) each time an electrical excitation signal (Ssrc(t)) is emitted by the audio communication unit (30) to said loudspeaker;
- a step E5 of determining the position of the microphone (M) implemented by the measuring device (40) when the coordinates of at least two spheres centred on different loudspeakers (HP) have been calculated, and in which the measuring device (40) calculates the points of intersection of the spheres, said points of intersection being possible positions of the microphone and being representative of the possible positions of the head (T) of the pilot when the latter uses his headset;
- a step E6 of measuring the movement of the microphone (M), in which the measuring device (40) measures, for a predetermined duration, the movements of at least one possible position of the microphone (M) termed the detection position, determined in the preceding step E5;
- a step E7 of determining lack of vigilance, in which the measuring device (40) determines that the pilot exhibits a lack of vigilance if the microphone (M) has not departed from the detection position by a predetermined distance for the predetermined duration;
- a step E8 of alert, implemented in the case where the measuring device (40) has determined that the pilot exhibits a lack of vigilance, in which the measuring device (40) generates an electrical signal (Salert) indicative of a lack of vigilance;
in the step E4 of calculation implemented for a loudspeaker (HP), the measuring device (40):
a) calculates the maximum of the intercorrelation function (*R_{SsrcSmic}*) between the electrical excitation signal (Ssrc(t)) of the audio communication unit (30) and the electrical signal (Smic(t)) originating from the microphone (M), the maximum thus calculated representing the acoustic propagation time of the electrical excitation signal;
b) calculates the distance from the loudspeaker (HP) to the microphone (M); and
c) determines the coordinates of the sphere around the loudspeaker (HP).

2. Method of detection according to Claim 1, **characterized in that** following the alert step E8, the electrical signal (Salert) indicative of a lack of vigilance of the pilot is transmitted to the audio communication unit (30), and **in that** on receipt of said electrical signal (Salert), the audio communication unit (30) emits an electrical excitation signal to at least one earpiece (31, 32) of the headset (C) for the emission of an alarm sound audible by the pilot.

3. Method of detection according to Claim 1, the aircraft (10) comprising an alert system (50) configured, in the case of the arising of an anomaly, to emit electrical alert signals and display alert messages on said at least one screen (21), **characterized in that** following the alert step E8, the electrical signal (Salert) indicative of a lack of vigilance of the pilot is transmitted to the alert system (50) for the displaying, on the at least one screen (21), of a message requesting the pilot to perform an action via the man-machine interface (22) within a predetermined time span, and
- if a pilot accomplishes the action within the time span, the measuring device (40) loops back to the step (E5) of determining the position of the microphone (M)
- if a pilot does not accomplish the action within the time span, a signal is dispatched by the measuring device (40) to an assistance on the ground for the implementation of assistance measures.

4. Method of detection according to any one of Claims 1 to 3, the headset (C) comprising a headband (F) intended to clasp the head (T) of the pilot when the headset is used, and a proximity sensor (33) mounted on the headband (F), a zone of detection of said sensor (33) being oriented towards a concave part of said headband, the sensor (C) being configured to generate a signal (Sactiv) comprising the information according to which the headset (C) is used when the head (T) of the pilot is situated within a predetermined distance from the sensor (33),
**characterized in that** the method comprises a step EP of verifying the use of the headset (C) by the pilot, implemented permanently by the measuring device (40), in which the measuring device (40) determines whether the headset (C) is used or not by the pilot as a function of the information contained in the signal (Sactiv) generated by the sensor (33),
and **in that** the alert step E8 is implemented on the further condition that the headset (C) is used by the pilot.

5. Method of detection according to any one of Claims 1 to 4, the aircraft (10) comprising an alert system (50) configured, in the case of the arising of an anomaly, to emit electrical alert signals and to display alert messages on said at least one screen (21), **characterized in that** an electrical excitation signal emitted by the audio communication unit towards a loudspeaker is a signal taken from among:
- a signal received from outside the aircraft (10);
- an electrical alert signal generated by the alert system (50);
- a signal specific to the detection of the incapacity of a pilot, generated by the measuring device (40).
